# EUROPEAN PATENT SPECIFICATION

(11) **EP 3 562 521 B1**
(45) Date of publication and mention of the grant of the patent: **03.05.2023**
(21) Application number: 17842342.2
(22) Date of filing: 21.12.2017
(51) Int. Cl.: A61L 27/00, A61L 27/02, A61L 27/12, A61L 27/46, A61L 27/56

(54) **METHOD OF MANUFACTURING COMPOSITE BONE IMPLANTS**
VERFAHREN ZUR HERSTELLUNG VON VERBUNDKNOCHENIMPLANTATEN
PROCÉDÉ DE FABRICATION D'IMPLANTS OSSEUX COMPOSITES

(30) Priority: 30.12.2016 PL 42005716
(43) Date of publication of application: 06.11.2019
(73) Proprietor: Instytut Wysokich Cisnien Polskiej Akademii Nauk, 01-142 Warszawa (PL)
(72) Inventor: LOJKOWSKI, Witold, 02-792 Warszawa (PL); CHUDOBA, Tadeusz, 01-318 Warszawa (PL); PIETRZYKOWSKA, Elzbieta, 07-130 Lochów (PL); MUKHOVSKYI, Roman, 01-875 Warszawa (PL); DABROWSKA, Sylwia, 18-208 Kulesze Koscielne (PL); CHODARA, Agnieszka, 03-101 Warszawa (PL); LOCS, Janis, 1007 Riga (LV); KROPIWNICKI, Jacek, 15-680 Bialystok (PL); CHARKIEWICZ, Michal, 16-061 Juchnowiec Koscielny (PL)
(74) Representative: Adamczyk, Piotr
(86) International application number: PCT/PL2017/050068
(87) International publication number: WO 2018/124897

(56) References cited:
- WO-A1-2006/015316
- KR-B1- 101 678 956
- US-A1- 2003 135 283

## Description

The invention regards a method of manufacturing composite bone implants containing synthetic hydroxyapatite.

A bone implant consisting entirely of synthetic hydroxyapatite has a high biocompatibility and bioactivity, but at the same time it has low impact strength and too high Young's modulus. Therefore, a composite consisting of crystalline hydroxyapatite and a polymer, e.g. bioresorbable polylactide (PLA), is often used to make such implants.

The optimal material for bone implant should be biocompatible (i.e. safe for the organism), have osteoconductive, osteoinductive and osteogenic properties, adequate biomechanical strength, be biodegradable, easy to dissect and implant, and be sterilizable. The autologous bone graft is considered the golden standard in the treatment of bone defects. The material for this purpose is taken from various parts of the patient's body, and the transplanted fragment of bone has all the properties needed to heal the damaged bone. However, this is not an ideal way to treat bone tissue. Limiting factors include, among others, a limited amount of tissue that can be removed, generalized disease process of the patient, anatomical operational limitations and excessive bone resorption during healing. Allografts (from another patient) or xenografts (from another species) are also used. Another method for bone regeneration is the use of synthetic (alloplastic) materials. In recent years, studies have been carried out on the biological, chemical and physical properties of synthetic materials for bone tissue remineralization. The influence of mechanical properties and microstructure on the regeneration process of bone tissue was also examined. Currently, metal, ceramic and polymer materials are most commonly used for this purpose.

An example of a composite hydroxyapatite and PLA implant has been disclosed in J.Russias et al. "Fabrication and mechanical properties of PLA/HA composites: A study of in vitro degradation" (Materials Science and Engineering: C, Vol. 26, I2006, pp. 1289-1295). This composite was obtained by chemical method, by dissolving polylactide in methylene chloride and adding microstructured hydroxyapatite ceramic particles, which were subjected to calcination at 1100°C for one hour. After mixing these components, a mass was obtained from which the fittings were made by casting in a Teflon form. These fittings were dried at room temperature for 24 hours to remove the solvent remains and then subjected to axial compression for 15 minutes at 45MPa and 220°C. Composites containing 50 to 90% of ceramic particles and bending strength from 65 to 125 MPa have been obtained this way.

In the publication of Sandrine Gay et al. "Preparation and characterization of dense nanohydroxyapatite/PLLA composites" (Materials Science and Engineering: C, Volume 29, Issue 1, 1 January 2009, pp. 172-177) the preparation of hydroxyapatite (HAP) composites by chemical method has been also disclosed. In this case chloroform was used as a solvent of the polymer and HAP with a particle size of 100 nm was used as the second component. The dissolved polymer with HAP particles was cast into a thin layer, which was cut into small pieces and placed in an axial compression mould. Compression was carried out at 160°C and pressure of 300 MPa. The influence of the HAP fraction on the mechanical properties of the composite has been examined. The increase in the amount of hydroxyapatite results in the increase of the compressive strength of the material and Young's modulus, but also its brittleness in comparison to pure ceramics. At 50% of the HAP content in the composite, a compressive strength of 100 MPa and a Young's modulus of 6 GPa were obtained.

The production of implants by dissolving biodegradable polymers in a solvent, e.g. in acetone or ethyl acetate, mixing with ceramic molecules, and then casting in the form has been also disclosed in US 5397572 and US 5492697.

In the publication of Artoum Rakovsky et al. "Strong bioresorbable Ca phosphate-PLA nanocomposites with uniform phase distribution by attrition milling and high pressure consolidation" [Journal of the Mechanical Behavior of Biomedical Materials, Volume 18, February 2013, pp. 37-46] a composite of HAP and PLA has been disclosed, obtained in the result of milling the ingredients in hexane in an argon atmosphere. The resulting powder was subsequently calcined at 700°C for 24 hours to obtain the β-TCP, Ca₃(PO₄)₂. Extending of the milling time to 24 hours allowed researchers to obtain material with high homogeneity, compressive strength of 400 MPa and Young's modulus of 10 GPa.

WO 2015/135822 A1 discloses a bioactive polymer of composite matrix with calcium phosphate particles, inter alia hydroxyapatite. This composite can have a form of gel, hydrogel or paste and is used as an injection filling. It contains solid particles with a size ranging from 50 nm to 10 µm. A similar solution has been disclosed in US 9333080 B2. The composite described therein contains 30% of biocompatible polymer and 70% of ceramics, and is in a form of a mass for injection, having mechanical properties similar to natural bone. The particle size of the ceramic component ranges from 10 µm to 1000 µm.

US 7875342 B2 and US 7052710 B2 disclose a method for obtaining porous implants in which porous ceramic sinters are prepared in the first step of the method. The sinters are then impregnated with the dissolved polymer. The implants obtained in this way have a material density in the range of 50 to 80%, and the pore size from 1 µm to 600 µm, with an average pore size in the range of 5 µm to 400 µm.

WO 2004/067052 A1 describes a production of bioabsorbable implants by injection of a bioabsorbable polymer and bioactive ceramic filler, i.e. hydroxyapatite. As a bioabsorbable polymer a polylactide, polyglycolide, polydioxanone, polycaprolactone, their copolymers and/or their mixtures were used. Injection moulding into a metal mould requires heating the mixture to a temperature of approx. 200°C.

US 2003/135283 A1 discloses a mixing and compression process for the manufacture of composite bone implants comprising nano-hydroxyapatite and PLA-fibers.

The aim of the invention was to create an implant made of bioresorbable materials suitable for use in orthopedics and stimulating the body to regenerate the bone tissue.

This aim is reached by implementing a method of manufacturing implants acccording to the invention comprising a step of preparation of a mixture of hydroxyapatite and polylactide and a step of forming an implant from the obtained mixture. The invention lies in the fact that in the step of mixture preparation, a synthetic hydroxyapatite with a particle size not exceeding 50 nm is mixed with a granulated polylactide with a particle size not exceeding 0.5 mm. The proportion of hydroxyapatite in the mixture is from 60% to 90% by weight. Next, the obtained mixture is milled cryogenically at a temperature no higher than -150°C and the obtained granulate is dried at a temperature no higher than 100°C.

In one variant of the invention a hydroxyapatite is used with a water content in the range of 1.5% to 5% determined by thermogravuret method at a temperature of 200°C.

In a further variant of the invention a hydroxyapatite is used with a molar ratio of calcium to phosphorus in the range of 1.57 to 1.61.

In a further variant of the invention the cryogenic milling lasts from 4 to 12 minutes.

In a further variant of the invention, in the step of implant formation, the dried granulate is placed in sealed elastic mould and compacted under pressure not less than 25 MPa and not more than 200 Mpa.

In a further variant of the invention a hydrostatic pressure is used for pressing.

In another variant of the invention the compacting under pressure is performed at a temperature ranging from 50°C to 200°C.

In yet another variant of the method of manufacturing implants according to the invention in the step of forming implant a rubber mould and a hydrostatic medium in form of a methyl silicone oil is used.

The invention allows one to obtain bone implants with a microstructure that stimulates the body to regenerate and that have mechanical properties similar to natural bone. The components of the implant material of the invention are bioresorbable, polylactide is widely used in medicine, and the implant material itself is hydrophilic and stimulates the body to rebuild bone tissue. The invention facilitates manufacturing implants of complicated shapes, e.g. bone screws.

Examples of embodiments of the invention are shown on the drawings, in which Fig. 1 presents an image of the microstructure of the implant material of the first example made with a scanning electron microscope (SEM image), Fig.2 shows the SEM image of the implant of the second example. Fig.3 shows the pore distribution in the material of the implant of the second example, and Fig.4 and Fig.5 show the SEM images of the microstructure of the implant material of the third and fourth example, respectively.

The invention will be described in further detail in the following embodiments:

### Example 1

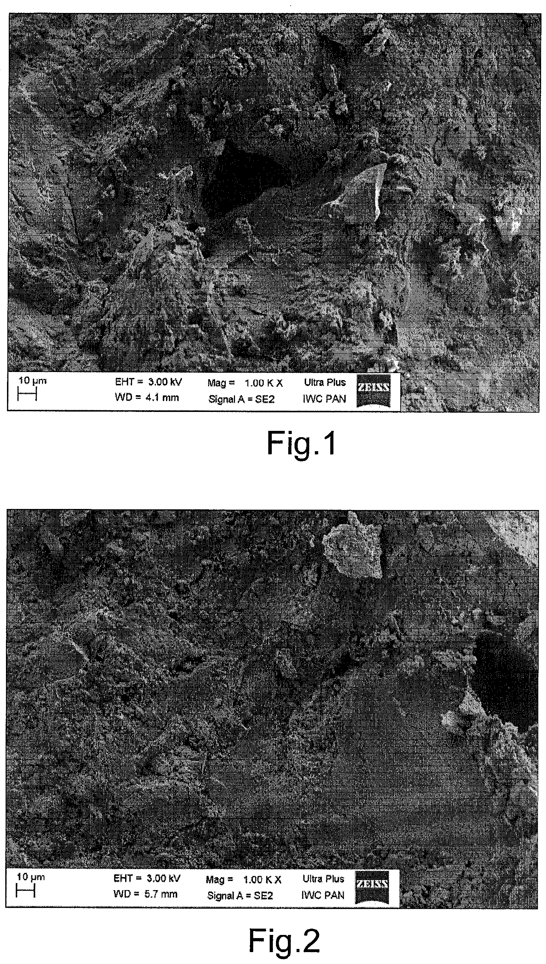

At the first stage a composite granulate was prepared from which an implant was then formed. In order to obtain said granulate, 2.8 g of synthetic hydroxyapatite were weighed, having particle size of not more than 50 nm and a molar ratio of calcium to phosphorus (Ca/P) of to 1.61. The hydroxyapatite was then dried, heated at a temperature raising from 100°C to 600°C for three hours with the rate of temperature rise of 2°C/min. After being cooled to the ambient temperature, a hydroxyapatite powder was obtained with a water content ranging from 1.5 to 5% as determined by thermogravimetry at a temperature of 200°C. A 1.2 g of commercial granulated polylactide with a density of 1.24 g/cm³, a relative viscosity of 3.3 and a particle size of about 0.5 mm was then weighed. This exact amounts of hydroxyapatite and polylactide were mixed at dry state and placed in a container of cryogenic mill type 6775 made by SPEX SamplePrep. In such mill an impact grinding of substances is possible, in a heat conducting container immersed in liquid nitrogen. In the container, apart from the substance being grinded, a ferromagnetic grinding impactor is placed, which is moved back and forth inside the container thanks to a variable magnetic field. The temperature in the said cryogenic mill was set at -190°C and the frequency of impacts of the grinding impactor was set at 12/s. The mill was turned on four times for two minutes, with intervals of five minutes for cooling the contents of the container to the starting temperature between successive periods of grinding. A composite granulate with grains of up to 100 µm was obtained, which was dried in vacuum and at temperature of 50°C for 12 hours. Then, two grams of dried composite granulate were weighed and placed in a tightly sealed rubber cylindrical form with a diameter of 14 mm and a volume of 2.31 cm³. The rubber mould was placed in a steel chamber, with capacity of 120 cm³, filled with methyl silicone oil and heated to a temperature of 155°C. After sealing the chamber a 50 MPa pressure was set and kept for 12 minutes. Thanks to this, the composite granulate in the rubber form was subjected to hydrostatic pressing, and after pressure release and cooling to room temperature, took a permanent shape of the mould. This resulted in a cylindrical body of 15 mm long and 8 mm in diameter, containing 30% by weight of polylactide and 70% of hydroxyapatite, with a characteristic microscopic structure visualized by means of a scanning electron microscope (SEM) and shown in Fig.1. Compressive strength of the obtained moulder was 110 MPa, its porosity determined by Archimedes method was 30%, and Young's modulus - 8 GPa.

### Example 2

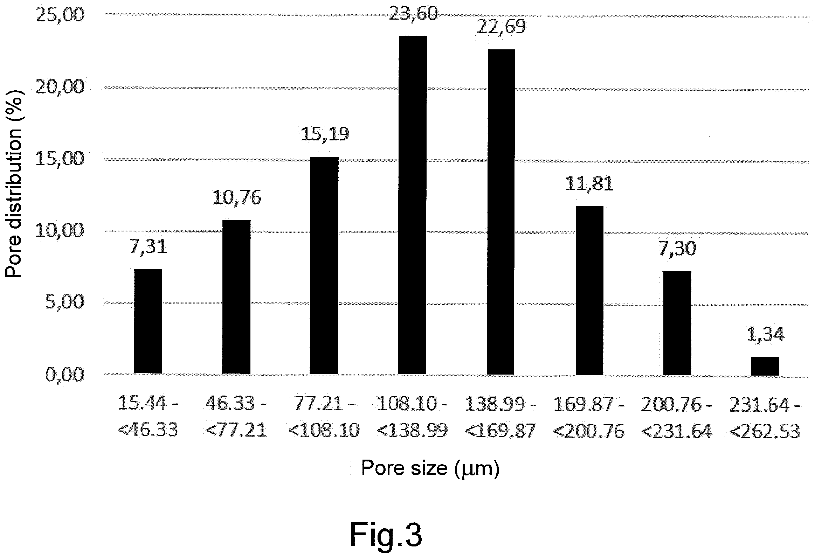

Similarly as in the first example, a mixture of hydroxyapatite with granulated polylactide was prepared, dried in the same manner as described above, containing 90% by weight of hydroxyapatite with particle size not exceeding 10 nm. The obtained mixture was grinded in the same cryogenic mill as before, the mill was turned on three times for two minutes, and the intervals between successive periods of grinding lasted 6 minutes. The obtained composite granulate was then dried in vacuum and at 100°C for 3 hours. Then, 2 grams of the dried composite granulate were weighed and placed in the same rubber form as in the first example and the same pressure chamber, the temperature of the methyl silicone oil of 165°C. After sealing the chamber, a pressure of 50 MPa was reached and kept for 12 minutes. This resulted in a cylindrical shape, 15 mm long and with 8 mm diameter, containing 10% by weight of polylactide and 90% of hydroxyapatite, with the microscopic microstructure shown in Fig. 2 and a density of 2.63 g/cm³. The compressive strength of the material of the obtained moulding was 80 MPa, and the bending strength - 26 MPa. The porosity of the resulting material, determined by means of computer tomography, was 1%, and the pore size distribution is shown in Fig.3.

### Example 3

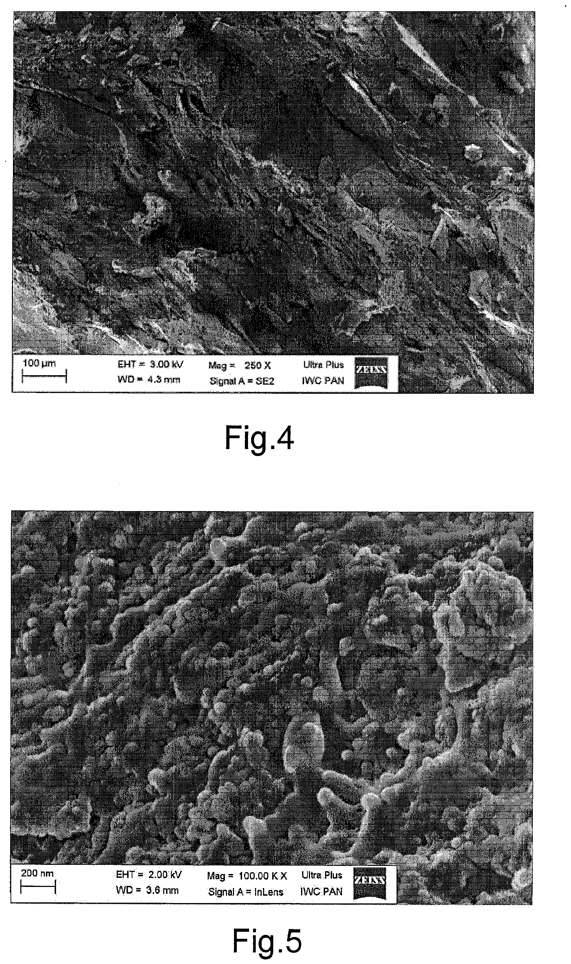

In the first stage, a composite granulate was prepared from which an implant was then formed. In order to obtain said granulate a 3.2 g of synthetic hydroxyapatite and 0.8 g of the commercial polylactide were weighed and dried as described in the first example. In order to obtain a composite granulate, similarly as in the first example, the polylactide and hydroxapatite were cryogenically grinded and the milling product was dried. From two grams of this composite granulate, a cylindrical implant was formed in a hydro-static manner as described in the first example,15 mm long and with 8 mm diameter, containing 20% by weight of polylactide and 80% of hydroxyapatite, with a density of 2.12 g/cm³, and the characteristic microstructure visualized with the scanning electron microscope (SEM) shown in Fig.4. The compressive strength of the material obtained was 100 MPa, its porosity determined by Archimedes method was 10%, and Young's modulus - 7 GPa.

### Example 4

At the beginning, a composite granulate with a granular size ranging from 50 µm to 500 µm containing 80% by weight of hydroxyapatite was prepared from the commercial polylactide and hydroxyapatite from the first example. This was done by dissolving the polylactide in a known solvent, mixing of the obtained solution with hydroxyapatite, then evaporating the solvent and grinding of the residue. Two grams of such composite granulate were subjected to hydrostatic moulding as described in the second example, i.e. at 165°C. This resulted in a cylindrical moulding 15 mm long and 8 mm in diameter, containing 20% by weight of polylactide and 80% of hydroxyapatite, with a microstructure visualized by a scanning electron microscope (SEM) shown in Fig.5. The density of the material of this moulding was 2.32 g/cm³ and the porosity was 2%, with the average pore size not exceeding 50 µm. Compressive strength of this material was 400 MPa, and bending strength - 40 MPa.

## Claims

1. A method of manufacturing composite bone implants, comprising a step of preparation of a mixture of hydroxyapatite and polylactide, and a step of forming an implant from the prepared mixture, **characterized in that** in the step of mixture preparation a dried synthetic hydroxyapatite having particle size not exceeding 50 nm is mixed with a granulated polylactide with a particle size not exceeding 0.5 mm, where the proportion of hydroxyapatite in the mixture is from 60% to 90% by weight, and the resulting mixture is cryogenically grinded at a temperature no higher than -150°C and the obtained granulate is dried at a temperature no higher than 100°C.

2. The method according to claim 1, **wherein** hydroxyapatite is used with water content from 1.5% do 5% determined by thermogravimetry at temperature of 200°C.

3. The method according to claim 1 or 2, **wherein** hydroxyapatite is used having molar ratio of calcium to phosphorus ranging from 1.57 to 1.62.

4. The method according to claim 1 or 2, **wherein** cryogenic grinding lasts from 4 to 12 minutes.

5. The method according to claim 1 or 2 or 3, **wherein** in the step of forming an implant the dried granulate is placed in tightly closed flexible mould and pressed with a pressure of not less than 25 MPa and no more than 200 MPa.

6. The method according to claim 5, **wherein** hydrostatic pressures are used for pressing.

7. The method according to claim 5 or 6, **wherein** pressing is performed in temperatures from 50°C to 200°C.

8. The method according to claim 7, **wherein** in the implant formation step a rubber form and a hydrostatic medium in the form of a methyl silicone oil is used.

## Patentansprüche

1. Verfahren zur Herstellung von Komposit-Knochenimplantaten, umfassend einen Schritt der Herstellung eines Gemischs aus Hydroxyapatit und Polylactid und einen Schritt der Bildung eines Implantats aus dem hergestellten Gemisch, **dadurch gekennzeichnet, dass im** Schritt der Herstellung des Gemischs ein getrocknetes synthetisches Hydroxyapatit mit einer Teilchengröße von nicht mehr als 50 nm mit einem granulierten Polylactid mit einer Teilchengröße von nicht mehr als 0,5 mm nicht überschreitet, wobei der Anteil an Hydroxyapatit in der Mischung 60 bis 90 Gew.- % beträgt, und die resultierende Mischung kryogenisch bei einer Temperatur von nicht mehr als -150°C gemahlen wird und das erhaltene Granulat bei einer Temperatur von nicht mehr als 100°C getrocknet wird.

2. Verfahren nach Anspruch 1, **wobei** Hydroxyapatit mit einem Wassergehalt von 1,5 % bis 5 %, bestimmt durch Thermogravimetrie bei einer Temperatur von 200°C, verwendet wird.

3. Verfahren nach Anspruch 1 oder 2, **wobei** Hydroxylapatit mit einem molaren Verhältnis von Calcium zu Phosphor im Bereich von 1,57 bis 1,62 verwendet wird.

4. Verfahren nach Anspruch 1 oder 2, **wobei** das kryogene Mahlen zwischen 4 und 12 Minuten dauert.

5. Verfahren nach Anspruch 1 oder 2 oder 3, **wobei** das getrocknete Granulat im Schritt der Bildung eines Implantats in eine dicht geschlossene flexible Form gegeben und mit einem Druck von nicht weniger als 25 MPa und nicht mehr als 200 MPa gepresst wird.

6. Verfahren nach Anspruch 5, **wobei** hydrostatische Drücke zum Pressen verwendet werden.

7. Verfahren nach Anspruch 5 oder 6, **wobei** das Pressen bei Temperaturen von 50°C bis 200°C durchgeführt wird.

8. Verfahren nach Anspruch 7, **wobei** in dem Schritt der Implantatbildung eine Gummiform und ein hydrostatisches Medium in Form eines Methylsilikonöls verwendet wird.

## Revendications

1. Procédé de fabrication d'implants osseux en matériaux composites, comprenant une étape de préparation d'un mélange d'hydroxyapatite et d'acide polylactique, et une étape de formation d'un implant à partir du mélange préparé, **caractérisé en ce que** dans l'étape de préparation du mélange, une hydroxyapatite synthétique séchée dont la taille des particules ne dépasse pas 50 nm est mélangée à un acide polylactique granulé dont la taille des particules ne dépasse pas 0,5 mm, la proportion d'hydroxyapatite dans le mélange étant comprise entre 60 % et 90 % en poids, et le mélange obtenu est broyé cryogéniquement à une température ne dépassant pas -150°C et les granules obtenus sont séchés à une température ne dépassant pas 100°C.

2. Procédé suivant la revendication 1, **dans lequel** l'hydroxyapatite utilisée a une teneur en eau de 1,5 % à 5 % déterminée par analyse thermogravimétrique à une température de 200°C.

3. Procédé suivant la revendication 1 ou 2, **dans lequel** l'hydroxyapatite utilisée présente un rapport molaire calcium/phosphore compris entre 1,57 et 1,62.

4. Procédé suivant la revendication 1 ou 2, **dans lequel** le broyage cryogénique dure de 4 à 12 minutes.

5. Procédé suivant la revendication 1 ou 2 ou 3, **dans lequel** lors de l'étape de formation d'un implant, les granules séchés sont placés dans un moule flexible fermé hermétiquement et pressés avec une pression d'au moins 25 MPa et d'au plus 200 MPa.

6. Procédé suivant la revendication 5, **dans lequel** la pression hydrostatique est utilisée pour la compression.

7. Procédé suivant la revendication 5 ou 6, **dans lequel** la compression est effectuée à des températures comprises entre 50°C et 200°C.

8. Procédé suivant la revendication 7, **dans lequel** l'étape de formation d'un implant utilise une forme en caoutchouc et un milieu hydrostatique sous la forme d'une huile de méthylsilicone.
